Europäisches Patentamt

European Patent Office

Office européen des brevets

(19)

(11) Publication number: 0 363 925
A1

# (12) EUROPEAN PATENT APPLICATION

(21) Application number: 89118885.6

(22) Date of filing: 11.10.89

(51) Int. Cl.⁵: **C07C 19/08 , C07C 17/38**

(30) Priority: 12.10.88 IT 2227388

(43) Date of publication of application:
18.04.90 Bulletin 90/16

(84) Designated Contracting States:
DE ES FR GB NL

(71) Applicant: AUSIMONT S.r.l.
31, Foro Buonaparte
I-20100 Milano(IT)

(72) Inventor: Gervasutti, Claudio, Dr.
2/4, Via P. Sarpi
I-30172 Mestre Venezia(IT)

(74) Representative: Barz, Peter, Dr. et al
Patentanwälte Dipl.-Ing. G. Dannenberg Dr.
P. Weinhold, Dr. D. Gudel Dipl.-Ing. S.
Schubert, Dr. P. Barz Siegfriedstrasse 8
D-8000 München 40(DE)

(54) Process for preparing 1,2-difluoro-1,1,2,2-tetrachloroethane substantially free of 1,1-difluoro-1,2,2,2-tetrachloroethane.

(57) A process for preparing 1,2-difluoro-1,1,2,2-tetrachloroethane substantially free of 1,1-difluoro-1,2,2,2-tetrachloroethane is described. Said process comprises the gas phase reaction of a mixture of said isomers with a slight molar excess of hydrogen in the presence of a catalyst comprising palladium metal supported on $Al_2O_3$, with contact times ranging from about 15 to about 25 seconds and at temperatures ranging from about 150° to 200°C.

EP 0 363 925 A1

# PROCESS FOR PREPARING 1,2-DIFLUORO-1,1,2,2-TETRACHLOROETHANE SUBSTANTIALLY FREE OF 1,1-DIFLUORO-1,2,2,2-TETRACHLOROETHANE

The present invention relates to a process for the (continuous) preparation of 1,2-difluoro-1,1,2,2-tetrachloroethane substantially free of the 1,1-difluoro-1,2,2,2-tetrachloroethane isomer.

1,2-Difluoro-1,1,2,2-tetrachloroethane is a quite interesting compound from an industrial point of view since it is utilizable either as such, for example in the field of solvents, or as intermediate for the preparation of halogenated olefins, such as fluoroethylenes and chlorofluoroethylenes (see, for example, Italian patent No. 1196518), or in the synthesis of perfluoroalkyl-vinylethers (IT-A-22348 A/86 and 22349 A/86) and of fluorinated hydrocarbons to be used, in admixture with other chlorofluorocarbons, as fluids for the Rankine cycle (IT-A-19077 A/88).

1,2-Difluoro-1,1,2,2-tetrachloroethane is a by-product of the production cycle of Algofrene®, 1,1,2-trifluoro-1,2,2-trichloroethane, and usually contains from about 8 to about 12% by weight of the isomeric 1,1-difluoro-1,2,2,2-tetrachloroethane.

Although said two difluorotetrachloroethane isomers exhibit chemical and physical characteristics different from one another, the techniques utilized so far for their separation (fractionated distillation and crystallization, precipitation from solvents, various chromatographic methods, selective chemical reactions, etc.) have proved to be not very effective, although said techniques are complicated and expensive.

Thus, it is an objection of the present invention to provide a simple and inexpensive process, which can be applied on an industrial scale and allows to separate the above isomers in relatively short times and in (large) amounts suitable for industrial utilization.

From the prior art (US-A-4,319,060) it is known to separate 1,2-dichloro-tetrafluoroethane from the 1,1-dichlorotetrafluoroethane isomer by treatment with hydrogen in the gas phase at various temperatures and in the presence of a dehydrochlorination catalyst. In practice, the preferred catalyst in said process is metallic palladium supported on carbon.

However this process, when applied to the separation of 1,2-difluoro-tetrachloroethane from the 1,1-difluoro-tetrachloroethane isomer, has proved to be not very effective because in the course of time 1,2-difluoro-tetrachloroethane (which does not participate in the reaction) deposits on the carbon surface (support), thereby blocking the active palladium-hydrogen exchange centres and causing a drastic reduction in the catalyst activity within short periods of time.

It has now, surprisingly, been found that by using a particular combination of operative parameters (catalyst support, metal, temperature, contact time etc.) it is possible to obtain 1,2-difluoro-1,1,2,2-tetrachloroethane substantially free of the 1,1-difluoro-1,2,2,2-tetrachloroethane isomer (the term "substantially free" means a purity of at least, and preferably higher than, about 99%, said purity being based on said two isomers only).

Thus, an object of the present invention is a process for the (continuous) preparation of 1,2-difluoro-1,1,2,2-tetrachloroethane substantially free of the 1,1-difluoro-1,2,2,2-tetrachloroethane isomer, said process comprising the gas phase reaction of a mixture of the above isomers with a slight molar excess of hydrogen, with respect to the isomer mixture, in the presence of a catalyst comprising metallic palladium supported on $Al_2O_3$, with contact times ranging from about 15 to about 25 seconds and at temperatures ranging from about 150 to about 200°C.

The isomer mixture utilized as starting material for the present process generally consists of a prevailing amount ( > 50%) of the 1,2-difluoro-1,1,2,2-tetrachloroethane isomer, which usually varies from about 75 to about 99% by weight, and of a smaller amount, generally from about 1 to about 25% by weight, of the 1,1-difluorotetrachloroethane isomer. The industrially produced 1,2-difluorotetrachloroethane usually contains from about 8 to about 12% by weight of the asymmetric isomer, and this mixture may advantageously be utilized as starting material for the present process.

According to the process of the invention, the mixture of the two isomers is reacted in the vapour phase with hydrogen in the presence of metallic palladium on alumina as catalyst.

The amount of supported palladium preferably ranges from about 0.1 to about 0.5%, based on the total weight of the catalyst.

Preferably, the alumina is utilized in the form of pellets and, prior to use, the catalyst is dried under vacuum for about 4 hours at about 120°C in order to remove moisture which would render the reaction less effective.

The reaction is conducted in the vapour phase and the amount of hydrogen is such that there is a slight molar excess (e.g. 10 to 30%) of hydrogen over the isomer mixture. Preferably, the molar ratio hydrogen/isomer mixture is about 1.2:1.

The process is conducted at temperatures ranging from about 150 to about 200°C. It was found that at temperatures lower than about 150°C low conversions (from 1 to 3%) are obtained and even no conversion at all can be observed when the temperature is below about 100°C.

On the other hand, when the process is conducted at temperatures higher than about 200°C, the conversion of 1,2-difluoro- 1,1,2,2-tetrachloroethane, in equal parts, to 1,1-difluoro-1,2,2,2-tetrachloroethane with prevailing formation of the unsaturated by-product $C_2Cl_2F_2$, is observed. Furthermore, the $C_2Cl_4F_2$ recovered at the end of the process still contains about 5% of 1,1-difluoro-1,2,2,2-tetrachloroethane.

The following examples are given in order to illustrate the present invention without being a limitation of embodiments thereof.

## EXAMPLE 1

0.50 Mols/h of a mixture, preheated to 100°C, of hydrogen and of difluoro-tetrachloroethane containing 12% by weight of 1,1-difluoro-tetrachloroethane (molar ratio $H_2$/isomer mixture = 1.2:1) were introduced, at atmospheric pressure, into a cylindrical steel reactor, thermoregulated at 150°C and having an inside diameter of 24 mm and a length of 275 mm and containing 105 ml of $Al_2O_3$ in the form of pellets with a palladium content of 0.2% by weight.
The contact time of the vapours of the reaction mixture and the catalyst was 20 seconds.
The vapours leaving the reactor were scrubbed and condensed in a 5% aqueous solution of NaOH to remove the hydrochloric acid contained in said vapours.
The organic portion, recovered by simple phase decantation and subjected to gas chromatographic analysis, exhibited the following composition:
1,2-difluoro-1,1,2,2-tetrachloroethane about 87.5% by weight
1,1-difluoro-1,2,2,2-tetrachloroethane about 0.5% by weight
1,1-difluoro-1,2,2-trichloroethane about 10.0% by weight
1,1-difluoro-dichloroethylene about 3.0% by weight.
Operating under the conditions specified in the example, after about 55 hours 2,900 g of isomer mixture had been fed to the reactor and no sign of decay in the catalytic activity could be observed.

## EXAMPLE 2 (comparative test)

Into the reactor employed in example 1, thermoregulated at 150°C and containing 105 ml of fresh carbon with a palladium content of 0.5% by weight, dried under vacuum at 100°C, there were introduced, at atmospheric pressure, 0.34 moles/h of a mixture, preheated to 100°C, of hydrogen and 1,2-difluoro-1,1,2,2-tetrachloroethane containing 12% by weight of 1,1-difluoro-1,2,2,2-tetrachloroethane, the molar ratio hydrogen/isomer mixture being 3:1.
The contact time of the vapours of the reaction mixture with the catalyst was 30 seconds.
Operating as in example 1, an organic mixture of general formula $C_2Cl_4F_2$ was recovered, which mixture contained only 1% by weight of asymmetric isomer.
However, when operating continuously, after about 20 hours the catalyst exhibited an activity loss due to saturation phenomena of the active centres, wherefore the results became unsatisfactory.

## EXAMPLE 3 (comparative test)

The reactor of example 1 was filled with pure and clean nickel chips (non-supported metal catalyst), and tests were carried out at different temperatures, i.e. at 200°, 300° and 400°C, feeding 0.0875 moles/h of $H_2$ and 0.049 moles/h of a difluorotetrachloroethane mixture containing 12% by weight of the 1,1-difluoro-1,2,2,2-tetrachloroethane isomer ($H_2$/isomer mixture molar ratio equal to 1.78:1).
Several runs were carried out and it could be observed that the conversion became higher with increasing temperature. However, the unconverted portion still contained about 8% by weight of asymmetric isomer.

## Claims

1. Process for the preparation of 1,2-difluoro-1,1,2,2-tetrachloroethane substantially free of 1,1-difluoro-1,2,2,2-tetrachloroethane, said process comprising the gas phase reaction of a mixture of the above isomers with hydrogen, in a slight molar excess with respect to said mixture, in the presence of a catalyst comprising palladium metal supported on $Al_2O_3$, with contact times ranging from about 15 to about 25 seconds and at temperatures ranging from about 150° to about 200°C.

2. Process according to claim 1, wherein the amount of palladium ranges from about 0.1 to about 0.5% by weight, based on the total catalyst.

3. Process according to any one of claims 1 and 2, wherein the palladium is present in an amount of about 0.2% by weight, based on the total catalyst.

4. Process according to any one of claims 1 to 3, wherein the contact time is about 20 seconds.

5. Process according to any one of claims 1 to 4, wherein the isomer mixture to be treated contains from about 75 to about 99% by weight of 1,2-difluoro-1,1,2,2-tetrachloroethane and from about 1 to about 25% by weight of 1,1-difluoro-1,2,2,2-tetrachloroethane.

6. Process according to any one of the preceding claims, wherein the isomer mixture to be treated contains from about 8 to about 12% by weight of 1,1-difluoro-1,2,2,2-tetrachloroethane.

7. Process according to any one of the preceding claims, wherein the molar ratio of hydrogen to isomer mixture is about 1.2:1.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | CHEMICAL ABSTRACTS, vol. 89, 1978, page 606, abstract no. 23750w, Columbus, Ohio, US; & CS-A-171 526 (V. DEDEK et al.) 15-02-1978 --- | | C 07 C 19/08 C 07 C 17/38 |
| A,D | US-A-4 319 060 (CUNNINGHAM et al.) ----- | | |

TECHNICAL FIELDS SEARCHED (Int. Cl.5)

C 07 C 17/00
C 07 C 19/00

The present search report has been drawn up for all claims

| Place of search | Date of completion of the search | Examiner |
|---|---|---|
| THE HAGUE | 05-01-1990 | VAN GEYT J.J.A. |

CATEGORY OF CITED DOCUMENTS ·

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)